# EUROPEAN PATENT APPLICATION

(11) **EP 0 707 065 A2**
(43) Date of publication of application: **17.04.1996**
(21) Application number: 95401754.7
(22) Date of filing: 24.07.1995
(51) Int. Cl.: C12N 15/29, C07K 14/415, A61K 39/36, C07K 16/16

(54) **Parietaria allergens**

(30) Priority: 22.07.1994 US 279113
(71) Applicant: UNIVERSITY OF MANITOBA, Winnipeg, Manitoba R3T 2N2 (CA)
(72) Inventor: Mohapatra, Shyam S., Winnipeg, Manitoba, R3P 2H1 (CA)
(74) Representative: Gutmann, Ernest

(57) **Abstract**

Screening of a Parietaria pollen-λgt22 library with antisera to a 14-kDa pollen protein from Parietaria led to the identification of several cDNA clones encoding allergenic proteins. Nucleotide sequence analysis of these clones indicated that, on the basis of the sequence homologies, these clones may be classified to three groups; group I (PJ001) comprising seven isoallergens, group II (PJ002) comprising two isoallergens and group III (PJ003) with three isoallergenic variants. Four of these cDNAs were expressed in pCNAII vectors which bound to the murine antibodies. IgE binding assay with 15-residue peptides designed from the deduced sequences of one of the group I proteins revealed a dominant IgE-binding region which spanned two overlapping peptides, peptides #27 and #28. These peptides are conserved among all of the group I iso-allergens. Furthermore, a murine monoclonal antibody directed to a major Parietaria- allergen bound to peptide #28, suggesting that this peptide is recognized by mouse antibodies as well. In a splenocyte culture of mice immunized with Parietaria allergens, peptide #28 stimulated the production cytokines IL-2, IL-4 and IFN-γ, whereas, peptide #23 stimulated production of only cytokine IFN-γ. Furthermore, Parietaria-specific antibodies were found in sera of North American subjects and these antibodies were shown to bind the 14 kDa Par j I allergen. Accordingly, these recombinant Parietaria allergens are useful in both diagnosis and therapy of allergic diseases induced by Parietaria pollens.

## Description

### FIELD OF INVENTION

The present invention is related to the field of molecular genetics and is particularly concerned with the cloning of the major allergens of the genus Parietaria.

This application is a continuation-in-part of United States Patent Application No. 08/279,113 filed July 22, 1994.

### BACKGROUND TO THE INVENTION

Pollen allergens are multideterminant proteins or glycoproteins capable of eliciting IgE-mediated allergic diseases, such as hayfever and asthma, in approximately 17% of the population who are genetically predisposed to develop allergies (ref. 1 - a list of the references appears at the end of the disclosure). In contrast to some other allergens (e.g., cat or house dust mite allergens), the global distribution of pollens of a large variety of monocotyledonous (grasses) and dicotyledonous plants (trees and weeds) preclude any realistic possibilities that individuals allergic to pollen allergens can avoid these aero-allergens.

Upon initial exposure to allergenic constituents, they are internalized by antigen-presenting cells (APC), which include nonantigen-specific phagocytic cells or specific B cells, and are "processed" by these cells. The net effect of this processing is the breakdown of the antigens into peptidic determinants which, in turn, are re-expressed in association with class I or class II molecules of the major histocompatibility complex (MHC) on the surface of the APC. Subsequently, the binary peptide-MHC complexes interact with the corresponding specific receptors (TCR) of, respectively, (i) Th cells or (ii) CTLs, and the resulting triads determine the up- or down-regulation of the appropriate B cells (ref. 2).

On the basis of their lymphokine secretion patterns, the Th cell subpopulation may be further subdivided into three subsets, i.e., Th0, Th1 and Th2 cells (ref. 3). In mice and man, the Th2 cells have been shown to produce IL-4, IL-5 and IL-6, and IL-4 has been shown to activate B cells leading to the production of IgE antibodies. In contrast, the Th1 cells produce IFNγ, which blocks the production of IL-4 and decreases IgE synthesis. Furthermore, IL-4 and IFNγ respectively induce and propagate Th2 and Th1 type of T-cell responses in both humans and mice (ref. 4).

The primary reaction of the IgE antibodies secreted from the Bε cells is their binding to specific IgE receptors on the surface of mast cells, basophils and eosinophils. On re-exposure of the patient to the specific multivalent allergen, the cell-fixed IgE antibodies react with and are crosslinked by the allergenic molecules. This process leads to the release from these cells of chemical mediators of anaphylaxis. In turn, these mediators act rapidly on the smooth muscles of different target organs, resulting in the inflammatory manifestations characteristic of immediate type hypersensitivity.

The development of therapeutic strategies (ref. 5) that may influence the formation of IgE antibodies requires detailed knowledge of (i) the structures of individual allergens and, in particular, of their B cell epitopes (which are recognized by IgE/IgG antibodies), and (ii) the structures recognized by T cell receptors of T cells, known as T cell epitopes. Therefore, determination of primary structures of individual pollen allergens of a complex repertoire of allergens of a given pollen by immunochemical and physicochemical methods is of central importance, and has been a major objective in allergy research for a long time.

The current treatment for hayfever consists primarily in symptomatic relief. Sufferers take drugs, such as anti-histamines and steroids, which do not suppress the formation of IgE antibodies and often have harmful side effects. Attempts to downregulate the IgE immune responses of allergic subjects by the "time-honoured" immunotherapy consist of a series of injections of increasing amounts of the allergenic extracts of the appropriate pollen or pollen-mixtures over prolonged periods lasting usually 3 to 5 years. Most of the pollen extracts used therapeutically are crude mixtures of a multiplicity of chemical constituents, some of these components bearing no relation whatsoever to the few allergenic components which are actually responsible for a given patient's hypersensitivity. Because some of the proteins present in these extracts may not be allergens, standardization of allergenic extracts based on total protein content is an unreliable guide for determining the potency of an extract. Moreover, large (up to 100x) variations in allergen content occur in the preparations used for immunotherapy because of the different methods used for (i) pollen collection and storage, which lead to variations in raw materials from lot to lot and from year to year, and (ii) the extraction procedures. Moreover, although different patients may be allergic to different constituents of a given pollen, all patients receive injections of the "same" complex mixture containing all the constituents of different pollens, i.e., they receive even components to which they may not be allergic. It is, therefore, not surprising that treatment with an ill-defined pollen extract may lead to the inauction of additional IgE antibodies, i.e., to sensitization of the patients to new components (refs. 6 to 10).

While up to 80% of patients gain clinical improvement from this immunotherapy (refs. 11, 12), the risk of side effects, the lengthy course of therapy, the inconvenience to the patient of the mode and frequency of administration, and the mounting costs of this treatment limit the utility of the current immunotherapy. Although, local and systemic reactions may occur as a result of this therapy, they may be managed by a physician specialized in allergy. However, occasionally this mode of treatment is associated with the risk of severe asthmatic or anaphylactic reactions, which can result in death (refs. 13, 14).

To eliminate some of the above disadvantages of the allergenic preparations currently used for immunotherapy, one of the major objectives in allergy research has been the isolation and characterization of the individual allergens of the complex repertoire of allergens of a given pollen by physicochemical and immunochemical methods. Several laboratories have isolated some of the allergens from the crude aqueous extracts of pollens by the use of classical physicochemical methods and reverse immunosorbents consisting of immobilized murine monoclonal antibodies to the pollen constituents (refs. 15, 16). Although such immunochemical methods appear to be promising for the characterization of individual allergens, the main drawback of these extremely labour intensive purification methods is the minute yield of allergens. Moreover, these methods do not ensure absolute purity of the allergenic constituents and, therefore, the determination of their amino acid sequences is difficult, if not impossible. As a corollary, the development of new therapeutic derivatives of pollen allergens and of reliable diagnostic procedures for pollen allergies are severely restricted by the use of allergens isolated by the existing procedures. However, recent innovations in recombinant DNA (rDNA) technology have paved the way for the synthesis of allergenic proteins, and of their epitopic fragments responsible for their activation of the appropriate B and T cells leading interactively to IgE formation, on an industrial scale and in a consistently pure state. Thus, recently some investigators have used the rDNA methods for the study of allergens. Accordingly, allergens present in dust mite (refs. 17 to 19), hornet venom (ref. 20), birch pollen (ref. 21) and grass pollens (ref. 22) have been cloned and the respective allergens produced by the application of rDNA techniques.

The pollens of plants belonging to genus Parietaria constitute a major source of aeroallergen and may account for up to 80% of the pollinosis in a number of countries world-wide (ref. 23). From the clinical allergy point of view, P. judaica and P. officinalis are the most relevant species. P. judaica is distributed in several regions of southern Europe including coastal districts of Spain, Southern France, Italy, Greece, Jugoslavia and Mediterranean Asia and Northern Africa. P. officinalis is distributed in Northern Italy, Central France and Central and Eastern Europe (ref. 24). Recently, the presence of allergy to this weed has also seen described in temperate climates of U.K. (ref. 25a) and U.S.A. (ref. 26b).

Although different forms of effective immunotherapy based on Parietaria extract have been reported (refs. 27a, 27b), all of these have some disadvantages including cost, compliance and though rare, severe anaphylactic reactions.

The allergenic constituents of the pollens of these species have been previously investigated. Both contain several allergens (refs. 25 to 34) and are cross-reactive (refs. 35, 36). The major allergens of P. judaica (Par j) have been reported by different groups, the reported molecular weight varies between 10 and 26 kDa (refs. 37 to 40). The allergens of P. officinalis (Par o) have also been described (refs. 40 to 43) and recently Par o I, a major glycoprotein allergen (size 14 kDa, pI 4.6) was reported. The amino acid composition and the sequence of the first 12 N-terminal residues were determined (ref. 44). In addition, recently a cDNA clone was reported for a major Par j allergen (ref. 44a).

### SUMMARY OF INVENTION

The present invention is directed toward improvements in allergen-specific immunotherapy by providing the means for overcoming the lack of pure allergens or peptides corresponding to allergenic portions thereof, as referred to above, by the determination of DNA sequences coding for allergenic proteins of Parietaria plant pollens. These groups of allergens, though constituting major allergenic components of Parietaria plant pollens, remained molecularly uncloned prior to this invention.

Accordingly, the present invention provides, in one aspect, a purified and isolated nucleic acid molecule comprising at least a portion coding for an allergenic protein which is present in pollens of Parietaria plants. Such Parietaria plants may be selected from the family Urticaceae, including P. judaica and P. officinalis.

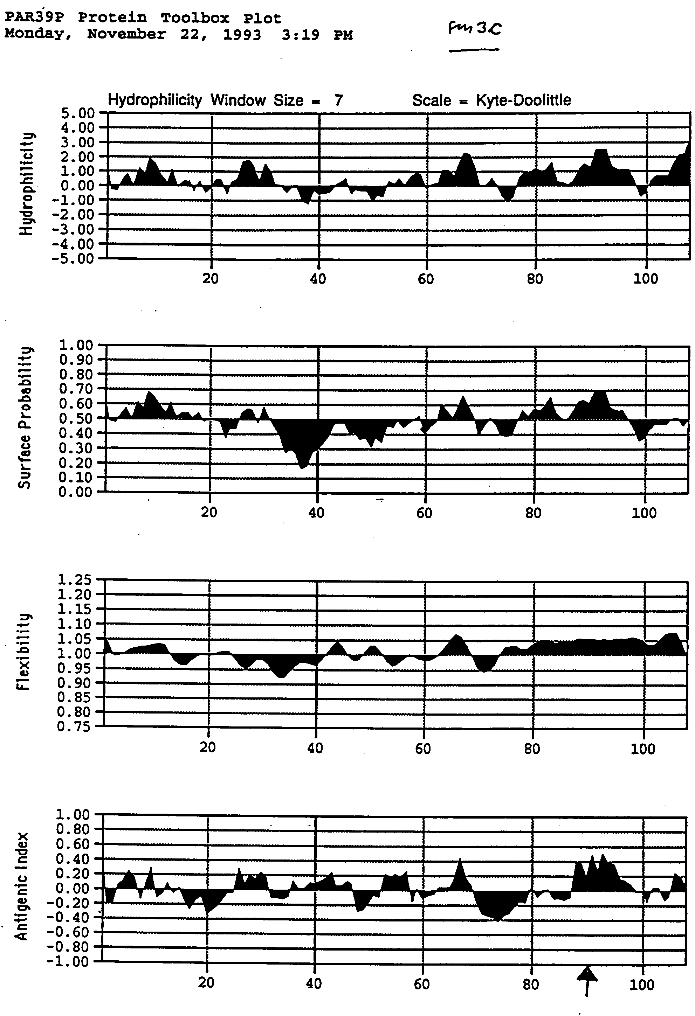

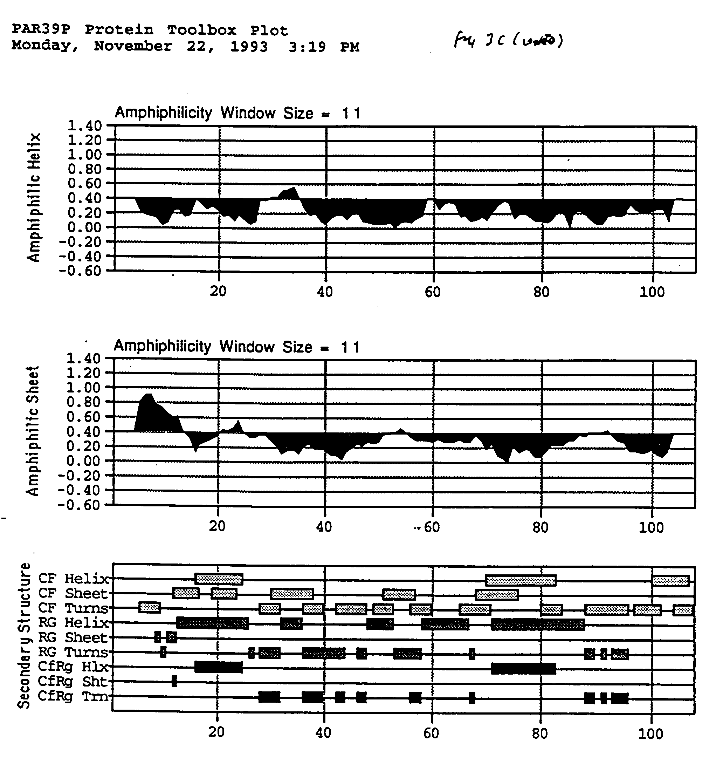

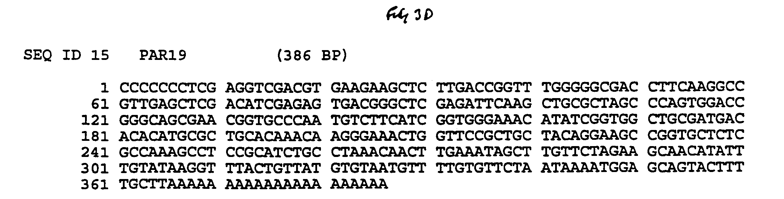

The portion of the nucleic acid molecule coding for the allergenic protein provided herein generally is selected from:
(a) the DNA molecule(s) including the DNA sequence(s) set out in Figure 3A (SEQ ID NOS 1 to 7), 3D (SEQ ID No: 15), 3F (SEQ ID NOS: 18, 19) or a complementary strand to any one of such sequences;
(b) the DNA molecule(s) encoding protein(s) including the amino acid sequence(s) set forth in Figure 3B (SEQ ID NOS: 8 to 14) or 3E (SEQ ID NOS 16, 17); and
(c) a DNA molecule(s) which hybridizes under stringent conditions to the DNA molecules of Parietaria allergens which may be conserved among those defined in (a) or (b). The DNA molecules defined in (c) have at least about 75% sequence identity with the DNA molecule(s) defined in (a) or (b). Figures 3A and 3B show the nucleotide sequences and amino acid sequences, respectively, for the PJ001 family, Figures 3D and 3E show these sequences for the PJ002 family and Figure 3F shows the nucleotide sequences for the PJ003 family of Parietaria allergens.

In another aspect of the invention, the present invention provides a recombinant plasmid adapted for transformation of a host, comprising a plasmid vector into which has been inserted a DNA segment comprising at least a 15 bp fragment of a nucleic acid molecule as provided herein. The present invention also includes an expression vector adapted for transformation of a host, comprising at least a DNA segment comprising at least a 15 bp fragment of a DNA molecule as provided herein and expression means operatively coupled to the DNA segment for expression thereof in a host. The DNA segment contained in the expression vector may further comprise a nucleic acid sequence encoding a carrier protein for expression of a carrier-allergen fusion.

An additional aspect of the invention provides a recombinant protein produced by expression in the host of the DNA fragment contained in the expression vector provided herein or a functional analog of the protein. In this application, a first protein is a "functional analog" of a second protein if the first protein is immunologically related to and/or has the same function as the second protein. The functional analog may be, for example, a fragment of the protein or a substitution addition or deletion mutant thereof. The present invention also includes synthetic allergenic or antigenic peptides corresponding in amino acid sequence to portions of the recombinant protein or allergen.

In a further aspect, the present invention provides a synthetic peptide having an amino acid sequence corresponding to at least one antigenic determinant of a Parietaria allergen. The Parietaria allergen may be selected from the PJ001 family, the PJ002 family and the PJ003 family. Such peptides may be selected from those shown in Table 2 (SEQ ID NOS: 21 to 35), in particular having the amino acid sequence FNKKFQSGKPPNEQL, YPLEKVTVEGNFYHK or NFYHKKCFRCFHGRC.

The present invention provides, in a further aspect, a composition for protecting allergic individuals from developing an allergic reaction, comprising at least one active component selected from at least one nucleic acid molecule, at least one recombinant protein, and at least one synthetic peptide, provided in accordance with aspects of the invention, and a pharmaceutically-acceptable carrier therefor, particularly formulated for in vivo administration.

In such immunogenic compositions, the composition may comprise at least one recombinant protein modified with a non-immunogenic substrate, for example, potassium cyanate (KCNO). The non-immunogenic substrate may comprise beads for targeted uptake of the at least one recombinant protein by antigen-presenting cells.

The composition may be formulated as a microparticle, capsule or liposome preparation and may be provided a combination with a targeting molecule for delivery to specific cells of the immune system or to mucosal surfaces. The composition provided in this aspect of the invention may be combined with at least one additional desensitizing agent, which may be selected from Poa p IX allergen, Lol p I allergen, Bet v I allergen, Amb a I allergen, Amb a II allergen and CRAL 51 allergen. In addition, the composition may comprise at least one compound having anti-histamine activity and/or at least one compound having anti-inflammatory activity and/or at least one compound which is immunosuppressive. The composition also may comprise an adjuvant.

In an additional aspect, the present invention provides a method for desensitizing an allergic individual, particularly a human, by administering an effective amount of the composition provided herein, particularly one containing the at least one additional desensitizing agent.

The present invention provides, in a further aspect, a method of depleting allergen-specific antibodies from an individual, particularly a human, by contacting the antibodies with the composition provided herein to form a complex, and removing the complex from the individual.

In another aspect, the present invention provides a method of anergizing allergen-specific antibody-producing cells, particular in a human, by contacting the cells with the composition provided herein.

In another aspect, the present invention provides a method of inducing Thi-like responses to Parietaria allergens by immunization with peptides inducing a predominantly IFNγ producing T cells.

An additional as part of the present invention provides a method for diagnosing an allergic reaction to pollens from Parietaria, which comprises administering to the individual the recombinant protein or peptide provided herein, and determining a response to the administration. An alternative diagnostic procedure comprises contacting serum from an individual with the recombinant protein or peptide, and determining the formation of a complex between the recombinant protein or the peptide and pollen specific antibodies in the serum.

The present invention also provides, in an additional aspect, an antiserum specific for a recombinant protein as provided herein.

### BRIEF DESCRIPTION OF DRAWINGS

Figure 1 shows a Western blot analysis of Parietaria pollen extract;
Figure 2 shows Western blot analysis of Parietaria phage clones during molecular cloning and identification of cDNA clones encoding Par j allergens;
Figure 3A shows the nucleotide sequences (SEQ ID NOS: 1 to 7) and Figure 3B shows the deduced amino acid sequences (SEQ ID NOS: 8 to 14) of Par j cDNA clones encoding Par j pollen proteins in group PJ001;
Figure 3C shows the protein structure analysis of Par 39P (SEQ ID NO: 13);
Figure 3D shows the nucleotide sequences (SEQ ID NO: 15) and Figure 3E shows the deduced amino acid sequences (SEQ ID NOS: 16, 17) of Par j cDNA clones encoding Par j pollen proteins in group PJ002;
Figure 3F shows the nucleotide sequence (SEQ ID NO: 18, 19) of Par j cDNA clones encoding Par j pollen proteins in group PJ003;
Figure 4 shows the expression cloning in *E coli* and reactivity of expressed protein with murine polyclonal antibodies to Par j I and human IgE antibodies. The recombinant proteins produced by E. coli were electrophoresed and reacted with murine polyclonal to purified Par j I. Lanes 1 to 5 contained proteins of clones Par 9, Par 39, Par 48, Par 64 and Par 70 respectively. Lane C contained proteins of E. coli containing the vector plasmid without insert. Murine polyclonal Ab to Par j I was used as the primary Ab;
Figure 5 shows the human antibody IgE binding of synthetic overlapping peptides by human sera;
Figure 6 shows the sequence alignment of the IgE binding peptide(s) shown shaded (SEQ ID NO: 7 to 28);
Figure 7 shows (a) the binding of peptide #28 to a murine monoclonal antibody specific to a Parietaria allergen, (b) ELISA inhibition of MAb binding to peptide #28 by Parietaria extract.
Figure 8 shows the occurrence of Parietaria IgE-specific antibodies in pollen-allergic subjects in North America; and
Figure 9 shows the Western blot analysis of Parietaria pollen extract using allergenic sera from Italy (A) and North America (B); and

### GENERAL DESCRIPTION OF INVENTION

It is clearly apparent to one skilled in the art, that the various embodiments of the present invention have many applications in the fields of diagnosis and therapy of allergic diseases, such as allergic rhinitis, asthma, food allergies and atopic eczema. In a diagnostic embodiment, the demonstrated IgE reactivity of the Parietaria recombinant allergen(s) is particularly useful. Thus, as described above, the usual method of determining to which aeroallergens an individual is allergic involves an intra-dermal exposure to crude extracts of Parietaria allergen extracts. This procedure involves the use and administration of many allergens. The recombinant Parietaria allergen(s) of the present invention now allow for the demonstration of IgE antibodies against a whole range of allergens. A further non-limiting discussion of such uses is further presented below.

### Preparation and Use of Compositions for Protecting Allergic Individuals from Developing an Allergic Reaction

Compositions, suitable to be used for protecting allergic individuals from developing an allergic reaction, may be prepared from recombinant Parietaria allergen(s), analogs, fragments and/or peptides as disclosed herein. Compositions may be prepared as injectables, as liquid solutions or emulsions. The recombinant Parietaria allergen fragment analogs or peptides may be mixed with pharmaceutically-acceptable excipients which are compatible with the allergen proteins, fragment analogs or peptides. Excipients may include, water, saline, dextrose, glycerol, ethanol, and combinations thereof. The composition may further contain minor amounts of auxiliary substances, such as wetting or emulsifying agents, pH buffering agents, or adjuvants to enhance the effectiveness thereof. Methods of achieving an adjuvant effect for the compositions includes the use of agents, such as aluminum hydroxide or phosphate (alum), commonly used as 0.05 to 0.1 percent solution in phosphate buffered saline. Compositions may be administered parenterally, by injection subcutaneously or intramuscularly. Alternatively, other modes of administration including suppositories and oral formulations may be desirable. For suppositories, binders and carriers may include, for example, polyalkylene glycols or triglycerides. Oral formulations may include normally employed incipients, such as, for example, pharmaceutical grades of saccharine, cellulose, magnesium carbonate and the like. These compositions take the form of solutions, suspensions, tablets, pills, capsules, sustained release formulations or powders and contain 10 to 95% of the allergen fragment analogs and/or peptides.

The compositions are administered in a manner compatible with the dosage formulation, and in such amount as is therapeutically effective to protect allergic individuals from developing an allergic reaction. The quantity to be administered depends on the subject to be treated, including, for example, the capacity of the individual's immune system to synthesize antibodies. Precise amounts of allergen required to be administered depend on the judgement of the practitioner. However, suitable dosage ranges are readily determinable by one skilled in the art and may be of the order of nanograms to micrograms of the allergen, analog, fragment and/or peptide. Suitable regimens for initial administration and booster doses are also variable, but may include an initial administration followed by subsequent administrations. The dosage of the composition may also depend on the route of administration and will vary according to the size of the host.

As described above, the Parietaria recombinant allergen may be modified by conjugating to a non-immunogenic substrate including polymeric materials such as carboxymethyl celluloses or potassium cyanate to render it non-allergenic for protecting allergic individuals from developing an allergic reaction (ref. 45). The availability of the recombinant Parietaria allergen in a purified form in large amounts now makes it possible to synthesize well defined conjugates for these molecules.

The nucleic acid molecules encoding the allergen of the present invention or portions thereof may also be used directly for immunization by adminstration of the DNA directly, for example, by injection for genetic immunization or by constructing a live vector, such as Salmonella, BCG, adenovirus, poxvirus, vaccinia or poliovirus. A discussion of some live vectors that have been used to carry heterologous antigens to the immune system are discussed in, for example, O'Hagan (ref. 46). Processes for the direct injection of DNA into test subjects for genetic immunization are described in, for example, Ulmer et al., 1993 (ref. 47).

The use of peptides corresponding to portions of the Parietaria recombinant allergen in vivo may first require their chemical modification, since the peptides themselves may not have a sufficiently long serum and/or tissue half-life. Such chemically modified peptides are referred to herein as a "peptoid". The term "peptoid" extends to any functional chemical equivalent of a peptide characterized by its increased stability and/or efficacy in vivo or in vitro in respect of the practice of the invention. The term "peptide analog" is also used herein to extend to any amino acid derivative of the peptides as described herein. Peptide analogs contemplated herein are produced by procedures that include, but are not limited to, modifications to side chains, incorporation of unnatural amino acids and/or their derivatives during peptide synthesis and the use of cross-linkers and other methods which impose conformational constraint on the peptides or their analogs.

Examples of side chain modifications contemplated by the present invention include modification of amino groups such as by reductive alkylation by reaction with an aldehyde followed by reduction with NaBH₄; amidation with methylacetimidate; acetylation with acetic anhydride; carbamylation of amino groups with cyanate; trinitrobenzylation of amino groups with 2, 4, 6, trinitrobenzene sulfonic acid (TNBS); alkylation of amino groups with succinic anhydride and tetrahydrophthalic anhydride; and pyridoxylation of lysine with pyridoxa-5'-phosphate followed by reduction with NaBH₄.

The guanidino group of arginine residues may be modified by the formation of heterocyclic condensation products with reagents, such as 2, 3-butanedione, phenylglyoxal and glyoxal.

The carboxyl group may be modified by carbodiimide activation via o-acylisourea formation followed by subsequent derivatisation, for example, to a corresponding amide.

Sulfhydryl groups may be modified by methods, such as carboxymethylation with iodoacetic acid or iodoacetamide; performic acid oxidation to cysteic acid; formation of mixed disulphides with other thiol compounds; reaction with maleimide; maleic anhydride or other substituted maleimide; formation of mercurial derivatives using 4-chloromercuribenzoate, 4-chloromercuriphenylsulfonic acid, phenylmercury chloride, 2-chloromercuric-4-nitrophenol and other mercurials; carbamylation with cyanate at alkaline pH.

Tryptophan residues may be modified by, for example, oxidation with N-bromosuccinimide or alkylation of the indole ring with 2-hydroxy-5-nitrobenzyl bromide or sulphonyl halides. Tryosine residues may be altered by nitration with tetranitromethane to form a 3-nitrotyrosine derivative.

Modification of the imidazole ring of a histidine residue may be accomplished by alkylation with iodoacetic acid derivatives or N-carbethoxylation with diethylpyrocarbonate.

Examples of incorporating unnatural amino acids and derivatives during peptide synthesis include, but are not limited to, use of norleucine, 4-amino butyric acid, 4-amino-3-hydroxy-5-phenylpentanoic acid, 6-aminohexanoic acid-, t-butylglycine, norvaline, phenylglycine, ornithine, sarcosine, 4-amino-3-hydroxy-6-methylheptanoic acid, 2-thienyl alanine and/or D-isomers of amino acids.

The purified and isolated DNA molecules comprising at least a portion coding for an allergenic protein which is present in Parietaria pollens typified by the embodiments described herein are advantageous as:
- nucleic acid probes for the specific identification of other allergens that contain a DNA sequence comprising at least a portion coding for the recombinant Parietaria allergen or analogs thereof;
- the products encoded by the DNA molecules are useful as diagnostic reagents to identify Parietaria specific IgE antibodies in an individual to demonstrate or exclude allergy to pollen allergens of a whole range of Parietaria and related plants;
- peptides corresponding to portions of the allergen as typified by the embodiments described herein are advantageous as diagnostic reagents, antigens for the production of allergen-specific antisera, for example, for the demonstration of an allergic reaction to a whole range of allergens.

### Immunoassays

The recombinant Parietaria allergen, analog, fragment and/or peptide of the present invention are useful as immunogens, as antigens in immunoassays including enzyme-linked immunosorbent assays (ELISA), RIAs and other non-enzyme linked antibody binding assays or procedures known in the art for the detection of allergen specific IgE antibodies. In ELISA assays, the allergen, analog fragment and/or peptides corresponding to portions of the allergen are immobilized onto a selected surface, for example, a surface exhibiting a protein affinity, such as the wells of a polystyrene microtiter plate. After washing to remove incompletely adsorbed allergen, analog fragment and/or peptides, a nonspecific protein, such as bovine serum albumin (BSA) or casein that is known to be antigenically neutral with regard to the test sample, may be bound to the selected surface. This allows for blocking of nonspecific adsorption sites on the immobilizing surface and thus reduces the background caused by nonspecific bindings of antisera onto the surface. Normally, the peptides are at least 12 residues in length and preferably 14 to 30 residues. It is understood however, that a mixture of peptides may be used either as an immunogen in a composition or as a diagnostic agent.

The immobilizing surface is then contacted with a sample, such as clinical or biological materials, to be tested in a manner conducive to immune complex (antigen/antibody) formation. This may include diluting the sample with diluents, such as solutions of BSA, bovine gamma globulin (BGG) and/or phosphate buffered saline (PBS)/Tween. The sample is then allowed to incubate for from about 2 to 4 hours, at temperatures, such as of the order of about 25° to 37°C. Following incubation, the sample-contacted surface is washed to remove non-immunocomplexed material. The washing procedure may include washing with a solution, such as PBS/Tween, or a borate buffer.

Following formation of specific immunocomplexes between the test sample and the bound allergen, analog, fragment and/or peptides, and subsequent washing, the occurrence, and even amount, of immunocomplex formation may be determined by subjecting the immunocomplex to a second antibody having specificity for the first antibody. If the test sample is of human origin, the second antibody would be an antibody having specificity for human IgE or IgG antibodies. To provide detecting means, the second antibody may have an associated activity, such as an enzymatic activity that will generate, for example, a color development upon incubating with an appropriate chromogenic substrate. Quantification may then achieved by measuring the degree of color generation using, for example, a visible spectra spectrophotometer.

### Use of Sequences as Hybridization Probes

The nucleotide sequences of the present invention, comprising the sequence of the Parietaria allergenic proteins, now allow for the identification and cloning of the allergenic protein genes from other Parietaria-related plants.

The nucleotide sequences comprising the sequence of the allergenic protein of the present invention are useful for their ability to selectively form duplex molecules with complementary stretches of other allergenic protein genes. Depending on the application, a variety of hybridization conditions may be employed to achieve varying degrees of selectivity of the probe toward the other allergenic protein genes. For a high degree of selectivity, relatively stringent conditions are used to form the duplexes, such as low salt and/or high temperature conditions, such as provided by 0.02 M to 0.15 M NaCl at temperatures of between about 50°C to 70°C. For some applications, less stringent hybridization conditions are required such as 0.15 M to 0.9 M salt, at temperatures ranging from between about 20°C to 55°C. Hybridization conditions can also be rendered more stringent by the addition of increasing amounts of formamide, to destabilize the hybrid duplex. Thus, particular hybridization conditions can be readily manipulated, and will generally be a method of choice depending on the desired results.

A wide variety of appropriate indicator means are known in the art for determining hybridization, including radioactive, enzymatic or other ligands, such as avidin/biotin, which are capable of providing a detectable signal. In some embodiments, an enzyme tag such as urease, alkaline phosphatase or peroxidase, instead of a radioactive tag may be used. In the case of enzyme tags, colorimetric indicator substrates are known which can be employed to provide a means visible to the human eye or spectrophotometrically, to identify specific hybridization with samples containing allergenic protein gene sequences.

The nucleic acid sequences of allergenic protein genes of the present invention are useful as hybridization probes in solution hybridizations and in embodiments employing solid-phase procedures. In embodiments involving solid phase procedures, the test DNA (or RNA) from samples, is adsorbed or otherwise affixed to a selected matrix or surface. The fixed, single-stranded nucleic acid is then subjected to specific hybridization with selected probes comprising the nucleic acid sequences of the allergenic protein genes or fragments thereof of the present invention under desired conditions. The selected conditions will depend on the particular circumstances based on the particular criteria required depending on, for example, on the G+C contents, type of target nucleic acid, source of nucleic acid, size of hybridization probe etc. Following washing of the hybridization surface so as to remove non-specifically bound probe molecules, specific hybridization is detected, or even quantified, by means of the label. The selected probe should be at least 18 bp and is preferably in the range of 30 bp to 90 bp long.

### Expression of the Allergenic Protein Genes

Plasmid vectors containing replicon and control sequences which are derived from species compatible with the host cell may be used for the expression of the allergenic protein genes in expression systems. The vector ordinarily carries a replication site, as well as marking sequences which are capable of providing phenotypic selection in transformed cells. For example, E. coli may be transformed using pBR322 which contains genes for ampicillin and tetracycline resistance and thus provides easy means for identifying transformed cells. The pBR322 plasmid, or other microbial plasmid or phage must also contain, or be modified to contain, promoters which can be used by the microbial organism for expression of its own proteins.

In addition, phage vectors containing replicon and control sequences that are compatible with the host microorganism can be used as a transforming vector in connection with these hosts. For example, the phage in lambda GEM™-11 may be utilized in making recombinant phage vectors which can be used to transform host cells, such as E. coli LE392.

Promoters commonly used in recombinant DNA construction include the β-lactamase (penicillinase) and lactose promoter systems (Chang et al., 1978 (ref. 48): Itakura et al., 1977 (ref. 49); Goeddel et al., 1979 (ref. 50); Goeddel et al., 1980 (ref. 51)) and other microbial promoters such as the T7 promoter system. Details concerning the nucleotide sequences of promoters are known, enabling a skilled worker to ligate them functionally with plasmid vectors. The particular promoter used will generally be a matter of choice depending upon the desired results. Hosts that are appropriate for expression of the allergen genes, fragment, analogs or variants thereof include E. coli, Bacillus species, fungi, yeast, higher eukaryotic cells, such as CHO cells, or the baculovirus expression system may be used.

In accordance with this invention, it is preferred to make the allergenic protein, fragment or analog by recombinant methods. Particularly desirable hosts for expression include Gram positive bacteria which do not have LPS and are, therefore, endotoxin free. Such hosts include species of Bacillus and may be particularly useful for the production of allergenic protein, fragments or analogs thereof.

The allergenic protein may also be produced as a fusion protein with, for example, glutathione-S-transferase, β-galactosidase and protein A.

The above disclosure generally describes the present invention. A more complete understanding can be obtained by reference to the following specific Examples. These Examples are described solely for purposes of illustration and are not intended to limit the scope of the invention. Although specific terms have been employed herein, such terms are intended in a descriptive sense and not for purposes of limitations. Immunological and recombinant DNA methods may not be explicitly described in this disclosure but are well within the scope of those skilled in the art.

### EXAMPLES

### Example 1:

This Example describes the production of murine polyclonal antibodies to Parietaria judaica allergen (Par j I).
Methods:

P. judaica pollen was supplied by Lofarma, Milan, Italy (purity 75%). The pollen proteins were extracted using 1:30 w/v of pollen to 0.125 mol/L NH₄ HCO₃ (pH 8.7) containing 0.05 w/v NaN₃ 1 mM phenyl-methyl sulphenyl fluoride (PMSF) to prevent proteolysis. The proteins were extracted from pollen overnight at 4°C under gentle stirring. The suspension was centrifuged at 1000 g for 20 min. 1 mg of pollen led to preparation of approximately 20 mg of proteins. The proteins were separated on 15% SDS-PAGE and protein components were visualized by staining of the gel with Coomasie blue. The 14 to 17 kDa band was excised from the unstained gel and the proteins obtained by further electrophoresis. This fraction is referred to as Par j I. The preparation was dialyzed in PBS and protein concentration was determined and aliquots were stored at -20°C.

Mouse antisera to PJ whole extract and to the SDS-PAGE purified Par j I component were produced in groups of BDF1 mice (4 mice each). Each animal received subcutaneous (s.c.) injection of 10 µg of protein preparation in alum. The mice were boosted at day 21 and day 50. The animals were bled 7 days after last injection and the sera were pooled and stored in aliquots of 1 ml at -20°C.

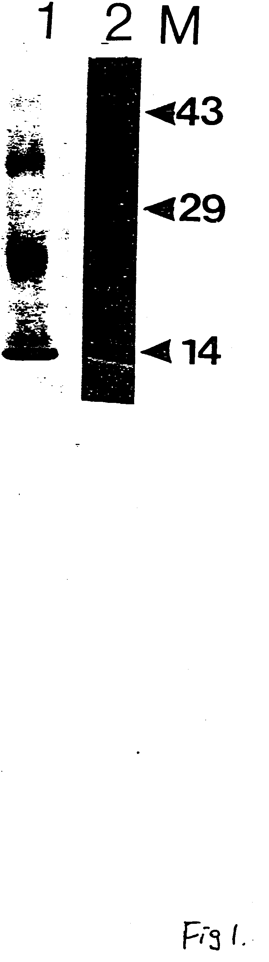

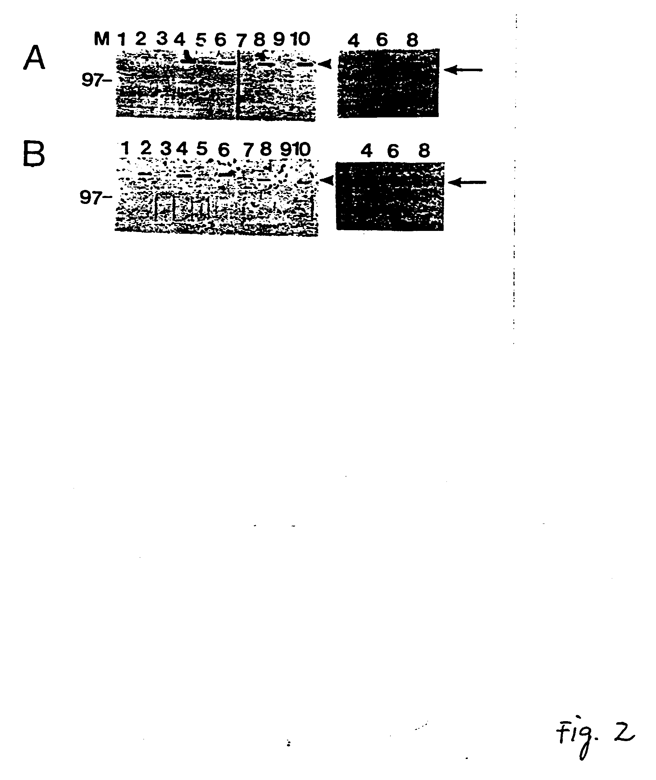

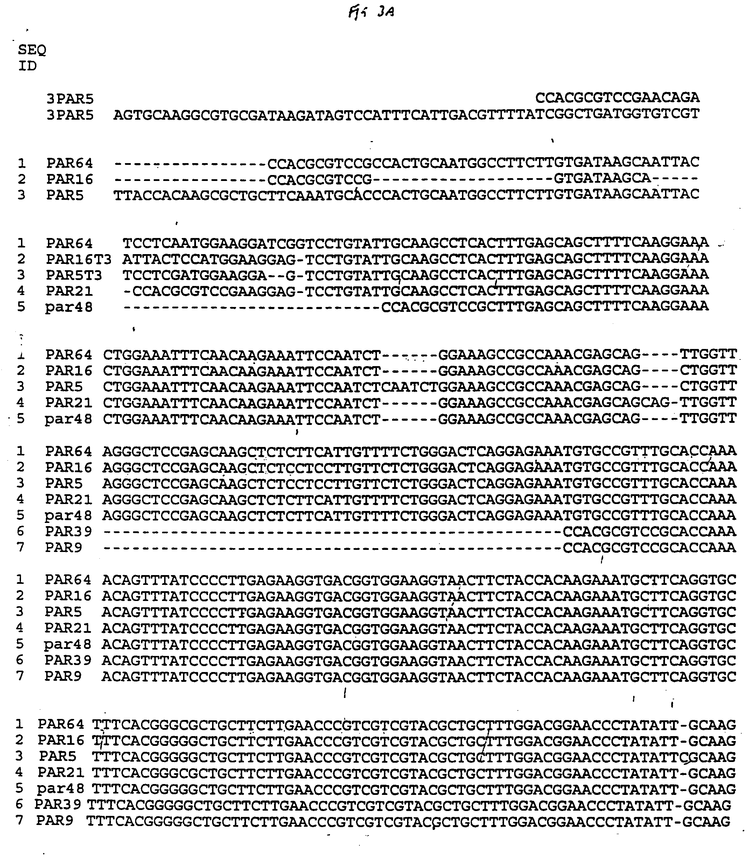

Figure 1 shows an Western blot of the Par j pollen extract proteins separated by SDS-PAGE. Following transfer of proteins, the nitrocellulose sheets were blocked with gelatin and then reacted either with murine, antiserum to Par j I or a pool of sera of 7 individuals allergic to Parietaria pollen. After overnight incubation at 4°C, the filters were washed and then incubated with an AP-conjugated appropriate second antibody. The antibody reactive proteins were detected by development of the membrane with substrate buffer.

The results demonstrate that the mouse antiserum recognized three polypeptide bands in this extract having molecular weight of about 14, 17 and 33 kDa. The human allergic serum pool also recognized two polypeptides of about 14 and 17.

It can be inferred from these results that the murine polyclonal antibodies and Human Serum IgE recognized the same proteins.

### Example 2:

This Example describes cDNA cloning of P. judaica pollen mRNAs, identification of cDNA clones and the reactivity of recombinant proteins with human IgE antibodies.

Total pollen RNA was extracted from 35 g of mature P. judaica pollen (Lofarma, S.R.L., Milan) using the guanidine isothyocyanate-cesium chloride method (ref. 52). Poly(a)+RNA was prepared by affinity chromatography on poly-U Sepharose (Pharmacia, Toronto). Approximately 10⁶ plaques from a λgt22 library were screened immunologically for the expression of pollen allergens using pooled immune-sera of mice immunized with Parietaria Par j I allergen. Plaques were lifted onto nitrocellulose filters (Bio-Rad, Toronto) and washed twice for 5 and 30 minute intervals in 30 mls of buffer (50 mM sodium phosphate, pH 7.5, 0.5% v/v Tween 20, 0.5% bovine serum albumin (BSA), 0.05% sodium azide w/v) per filter. The filters were then allowed to hybridize overnight at 4°C under slight agitation in buffer containing 0.01% pooled sera. The filters were then washed at 4°C, for three 15 minute intervals in 25 ml of buffer per filter, and incubated overnight at 15°C with goat-anti-mouse IgG conjugated with alkaline phosphatase. The filters were re-washed as previously described and developed using 5-bromo-4-chloro-3-indolylphosphate p-toluidine and nitroblue tetrazolium chloride. Positive clones were picked and re-screened, and further positive clones, were further characterized.

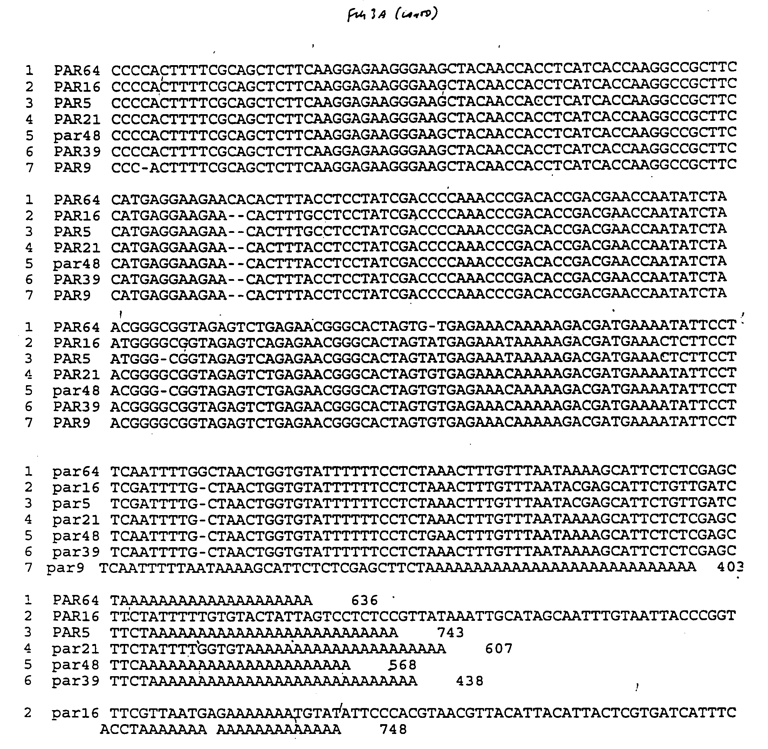

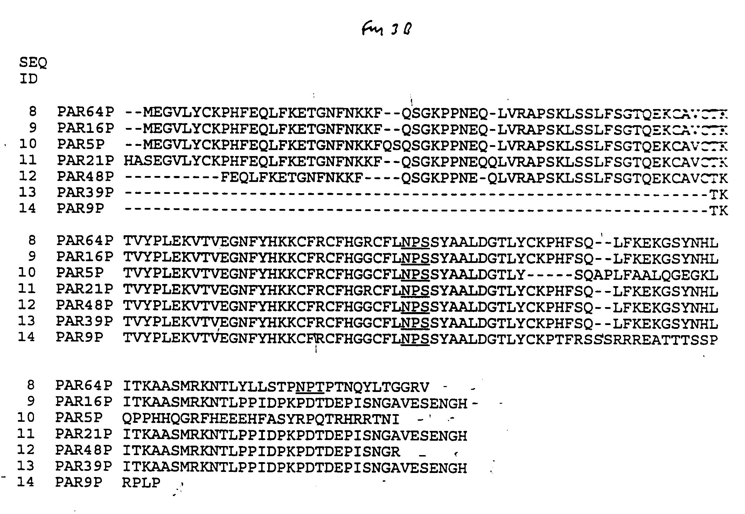

Proteins were isolated from recombinant phage clones and western blotted as described. Figure 2a shows lanes marked by numbers in brackets as follows: control (1,2); Par 9 (3,4); Par 39 (5,6); Par 48 (7,8); and Par 64 (9,10) isolated under non-induced (marked by odd numbers) and IPTG-induced (marked by even numbers) conditions. Western blot analysis was carried out using murine polyclonal Ab to Par j I. On the right panel is shown the Western blot of proteins from clones 4, 6 and 8 developed utilizing human allergic serum. M denotes the position of the marker protein and the arrowhead indicates the position of recombinant protein reacting with human IgE antibodies.

Figure 2b shows a Western blot of proteins isolated from phage clones on lanes marked by numbers in brackets as follows: Par 10 (1,2); Par 11 (3,4); Par 12 (5.6); Par 70 (7,8); and control (9,10) developed using murine polyclonal Ab to Par j I; the proteins were isolated under non-induced (lanes marked by odd numbers) and IPTG-induced (lanes marked by even numbers) conditions. On the right panel is shown the Western blot of lanes containing proteins of clones 4, 6, 8 which was developed utilizing human allergic serum as primary antibodies.

It can be inferred from these results that the Parietaria pollen proteins cloned are IgE binding proteins and correspond to major allergens of Parietaria.

### Example 3:

This Example describes the sequence analysis of cloned Parietaria allergens.

To determine the nucleotide sequences of the clones produced in Example 2, the cDNA inserts were excised by Not I-Sal I digestion, recloned in the pBluescript, and sequenced according to the procedures described previously (ref. 53). The ligation products were transformed in the E. coli strain DH5αF' lacIq⁻. The DNA was prepared from the recombinant transformants and DNA sequencing was performed employing dsDNA cycle sequencing system (BRL, Mississauga, Ont.) as per manufacturer's instructions. Briefly, 1 pmol of the T7 or SP6 primer, which flanked the poly linker of pBluescript, was end-labelled using [γ-³P] ATP (> 5,000 Ci/mmol) at 37°C for 30 min and 55°C for 5 min, template DNA (100-150 ng) in Taq sequencing buffer and 5 units of Taq DNA polymerase was added. The mixture was cooled on ice and distributed equally to four labelled tubes containing termination mix. Each tube was allowed to amplify for 20-30 cycles in the thermal cycler. The reaction products were electrophoresed on a 6% polyacrylamide/TBE-urea gel, which at the end of the run were dried and autoradiographed. Sequences were determined by sequencing both strands of DNA at least twice. DNA sequences were compiled and analysed by the Pustell sequence analysis program, BIRCH software package and protein analysis tool box.

All cDNAs possessed features typical of plant cDNA, for example, they had 3'-untranslated sequences which contained canonical AATTAAA polyadenylation signal followed by a poly A tail. On the basis of sequence analysis, the cDNAs were classified into three groups (Listed in Table 1 and Figure 3, which have been designated PJ001 Family, PJ002 Family and PJ003 Family). The nucleotide and the deduced amino acid sequences are shown in Figures 3A, 3B, 3D, 3E and 3F. Each sequence is identified by a clone number and its own sequence ID. Comparison of sequences revealed that the cDNA clones of each group have significant sequence homology, however they can be distinguished from each other by the sequence variations at the 3' ends.

The open reading frames were deduced for group I (PJ001) and group II (PJ002) allergens. Three of the cDNAs in group I appeared to be full-length clones and had a methionine start codon. The sequences were typically rich in leucine and lysine. There exist two potential glycosylation sites (N-P-T, underscored) which suggest that the encoded proteins are glycoproteins. The protein encoded by cDNAs Par 64, Par 16 and Par 5, respectively, are 19.1, 17.2 and 16.1 kDa and have pIs of 11.1, 10 and 10.5. These allergens appear to possess a signal peptide at the N-terminus. However, the precise cleavage sites are not known. For the group II allergens, reading frames without any stop codons could be deduced whereas this was not possible for group III (PJ003) allergens, suggesting that the sequence data for the PJ003 allergens may be considered preliminary at this point. Comparison of the nucleotide and deduced amino acid sequences set forth in Figures 3A, 3B, 3D, 3E and 3F with the GenBank DNA and PIR protein sequence Data Banks, indicated that the sequences were unique and not homologous to any other known allergen or non-allergen DNA or proteins in these Data Banks. Thus, the cloned cDNAs encode novel allergens which have not been described previously and are themselves novel.

### Example 4:

This Example describes the synthesis of the recombinant allergens in high level expression vectors.

DNA was prepared from plaque purified phage using the liquid lysate method (ref. 54). Inserts recovered by Not I-Sal I digestion were ligated into the multiple cloning site (MCS) of pcDNAII and the resulting plasmid used to transform E. coli DH5α.

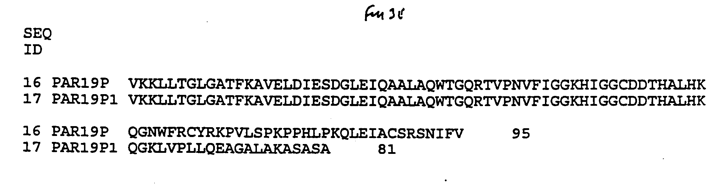

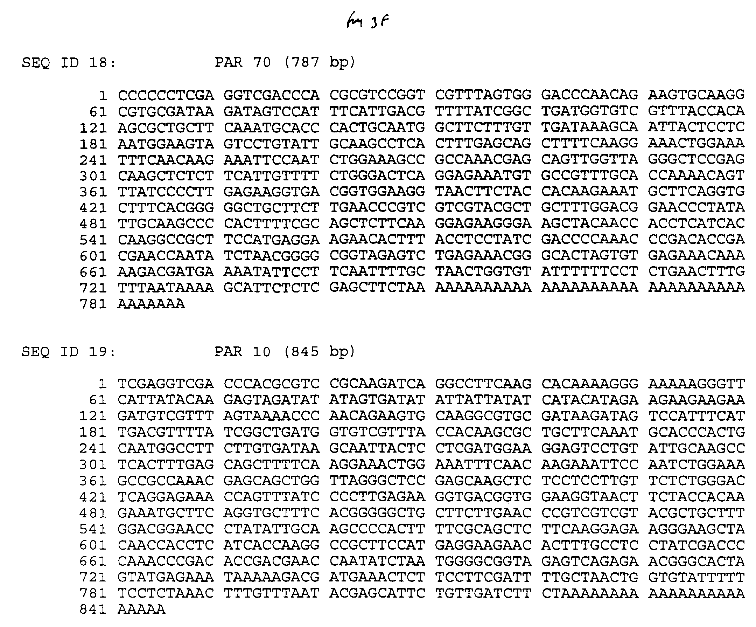

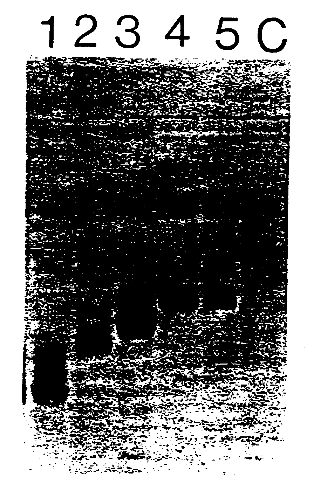

Figure 4 describes the Western blot analysis of the recombinant Parietaria proteins. The recombinant proteins produced by E. coli were electrophoresed and reacted with murine polyclonal antibody to purified 14 kDa Par j I. Lanes 1 to 5 contained proteins of clones Par 9, Par 39, Par 48, Par 64 and Par 70, respectively. Lane C contained proteins of E. coli containing the vector plasmid without insert. Murine polyclonal Ab to Par j I was used as the primary Ab.

It can be inferred from these results that the Parietaria recombinant allergens can be readily expressed utilizing other vectors and the corresponding recombinant allergens can be synthesized.

### Example 5:

This Example describes the determination of an IgE binding epitope of Par j I allergens.

To determine the antibody binding epitopes of Par64 allergen, a set of 15 overlapping peptides were synthesized (Table 2). The binding of these peptides to the pool of sera of five Parietaria allergic patients was examined by ELISA. Briefly, each well of Nunc maxisorb (BRL, CA) microtiter plate was coated with 10µg peptide in 100µl of 0.05M carbonate/bicarbonate buffer, pH9.6, overnight at 40°C. The plates were then washed with PBS-Tween buffer three times, and the free sites of the wells were saturated with 2% BSA in PBS buffer by incubation at room temperature for 2 hours. After washing, the wells were incubated with 100µl of the pooled human sera. For detection of human IgE-binding peptides, 1:2 diluted human sera were used. PBS containing 0.5% BSA and 0.1% Tween-20 was used as dilution buffer. The plates were incubated with the antisera at room temperature overnight, and washed three times with PBS-Tween buffer. The bound antibodies were detected with alkaline phosphatase conjugated goat anti-human IgE (TAGO, CA). All the second antibodies were used at 1:2000 dilution. The colour development of the substrate was carried out at 37°C for 60 minutes and the O.D. values were read with an ELISA reader. The O.D. values were regarded as positive when the reading was more than three folds of the negative control. Each ELISA was repeated at least twice.

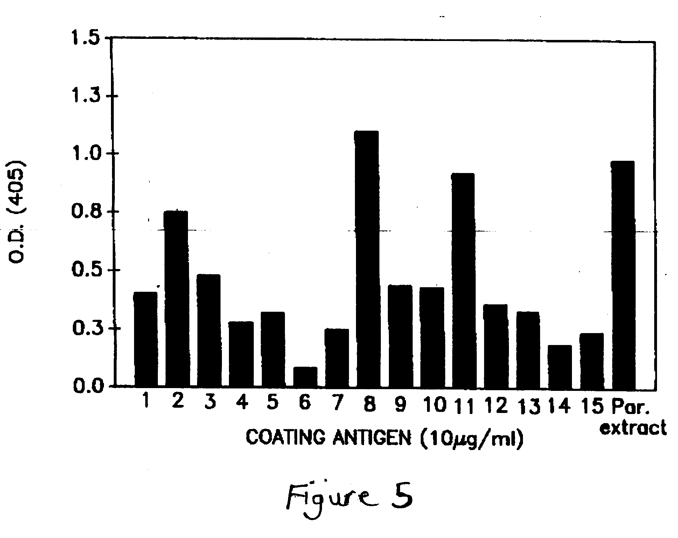

The results shown in Figure 5 demonstrate that peptide #28 bound to the human IgE antibodies. This binding was about two-fold that of control peptides.

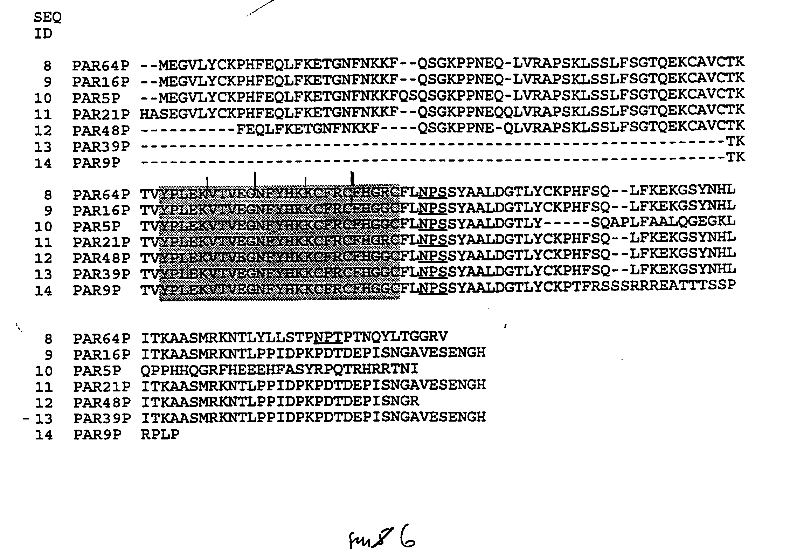

For a direct demonstration of human IgE binding to those peptides, a set of eight overlapping 15 residue peptides (with 5 residues overlapping) #21 to #28 were designed from the deduced amino acid sequence of Par 64 allergen. Ten microgram each of the peptides was dot-blotted onto nitrocellulose membrane, the membrane was blocked with BSA and then reacted overnight with a pool of sera of Parietaria allergic patients. The membrane was then washed and reacted with ¹⁵I labelled anti-human IgE antibodies and the membrane was autoradiographed. Only two peptides of eight examined, namely, peptides #27 and #28 reacted with the human IgE antibodies. It is inferred from these results that these two peptides include at least one IgE binding epitope of the Par 64 allergen. Furthermore, comparison of the sequences of these epitopes in other isoallergens (Figure 6) indicate that these epitopes are conserved also among other isoallergens of this group of Parietaria allergens.

### Example 6:

This Example describes the establishment of a murine, model of Parietaria allergy and identification of an antibody binding epitope and T cell epitopes on synthetic overlapping peptides of Par 64 allergen.

To examine if mice will induce IgE antibodies to Parietaria allergens, Parietaria extract-specific antibodies were induced in Balb/c and BDF1 mice by immunization of these mice with Parietaria extract (10 µg per mouse) in alum (1 mg per mouse) as adjuvant at day 1 and booster injections at day 21 with the same antigen. IgE antibody titers were determined as PCA titers in serum collected 7 days after booster. Hooded rats were sensitized with 100 µl of serially diluted mouse sera injected intradermally on the back. 48 hours later the rats were challenged i.v. with Parietaria extract, I mg in 1 ml PBS containing 0.5 % Evan's blue. PCA titer was calculated as the highest dilution giving a clearly visible skin reaction (>0.5 cm in diameter). Both strains of mouse exhibited detectable PCA titers (Table 3), Balb/c exhibited a higher PCA titer than that of BDF1 mice.

To determine the antibody binding epitopes of Par64 allergen, a set of 15 overlapping peptides were synthesized (Table 2). The binding of these peptides to the murine anti-Parietaria monoclonal antibody was examined by ELISA. Briefly, each well of Nunc Maxisorb (BRL, CA) microtiter plate was coated with 10 µg peptide in 100µl of 0.05M carbonate/bicarbonate buffer, pHg.6, overnight at 4°C. The plates were then washed with PBS-Tween buffer three times, and the free sites of the wells were saturated with 2% BSA in PBS buffer by incubation at room temperature for 2 hours. After washing, the wells were incubated with 100µl of the murine mAB raised against the Parietaria allergen. PBS containing 0.5% BSA and 0.1% Tween-20 was used as dilution buffer. The plates were incubated with the antisera at room temperature for two hours, and washed three times with PBS-Tween buffer. The bound antibodies were detected with alkaline phosphatase conjugated goat anti-mouse Ig (Zymed, CA) at 1:2000 dilution. The colour development of the substrate was carried out at 37°C for 60 minutes and the O.D. values were read with an ELISA reader. The O.D. values were regarded as positive when the reading was more than three folds of the negative control. Each ELISA was repeated at least two times.

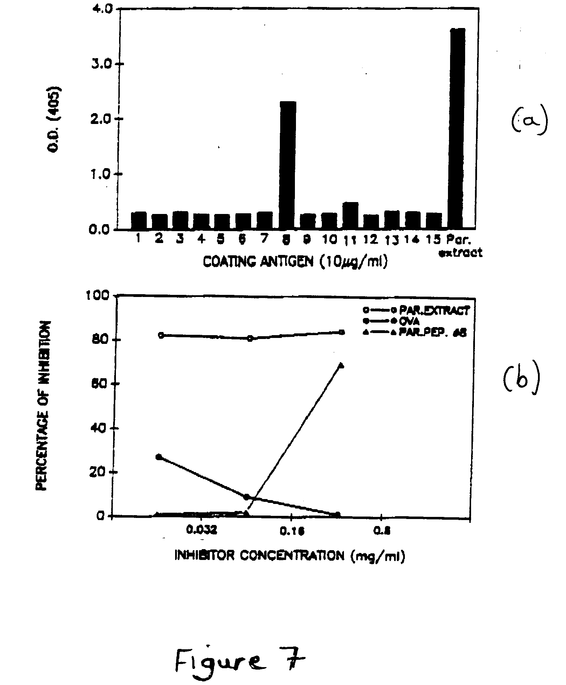

The results shown in Figure 7a demonstrate that peptide #28 bound to the murine monoclonal antibodies. This binding was about two-fold that of control peptides. To verify the authenticity of binding, an inhibition experiment was performed. Thus the pooled sera was incubated with increasing dose of Parietaria proteins and the absorbed sera was used for ELISA using peptide #28 as coated on the microtiter wells. The results in Figure 7b demonstrate that the binding to peptide #28 was inhibited in a dose dependent manner by incubation of sera with Parietaria allergens. The results demonstrate that peptide #28 contains also an epitope recognized by antibodies in mouse.

### Example 7:

This Example demonstrates identification of two T cell epitopes of the Par64 allergen which differ in their ability to stimulate cytokine production.

To identify the T cell epitopes of Par64 allergen, a set of 15 overlapping peptides were synthesized (Table 2). BDF1 mice were immunized with Parietaria extract in alum. Mouse spleens were collected aseptically on day 6 after first immunization. Single cell suspensions were prepared with a homogenizer, and debris were removed by passing cell suspensions through nylon membranes. Cells were spin down and washed once with RPMI 1640 medium containing 5% total calf serum, then cultured at number of 10 x 106/ml (2 ml/well) alone or with 200 µg/ml of peptide in 24 well tissue culture plate at 37°C in RPMI 1640 medium containing 10% fetal calf serum. Cells of individual spleens and pooled lymph nodes from each group were cultured for 24 hours, than supernatants were harvested and stored at -20°C until cytokine production was analysed. Cytokine production of IL-2, IFN-γ and IL-4 was determined with sandwich ELISA reagents (PharMingen, San Diego, CA). Briefly, 96 well ELISA plates were coated with 0.5 ug/well of purified anti-cytokine capture mAb overnight at 4°C, then blocked with 3% bovine serum albumin (BSA). Serially diluted recombinant cytokine standards and culture supernatants of spleen and lymph node cells were added and incubated overnight at 4°C. 1 ug/well of biotinylated anti-cytokine detecting MAb was added and detected with alkaline phosphatase conjugated streptavidin.

The results presented in Table 4 demonstrate that peptides differed in their capacity to produce cytokines measured, i.e., IL-2, IL-4 and IFN-γ. The results indicate that whereas peptides #23 stimulated primarily the production of IFN-γ, peptide #28 stimulated production of all cytokines including IL-4. It is referred that both of these peptides have the potential for use in immunotherapy.

### Example 8:

This Example describes the occurrence of Parietaria specific IgE antibodies in pollen allergic subjects in North America.

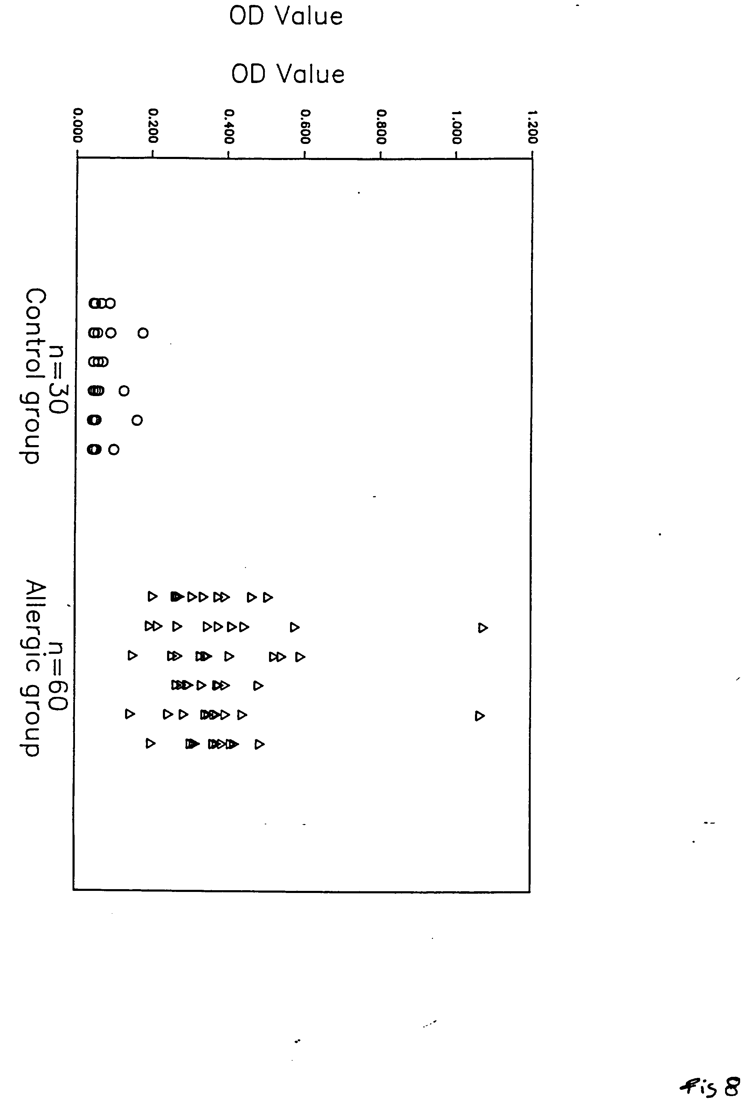

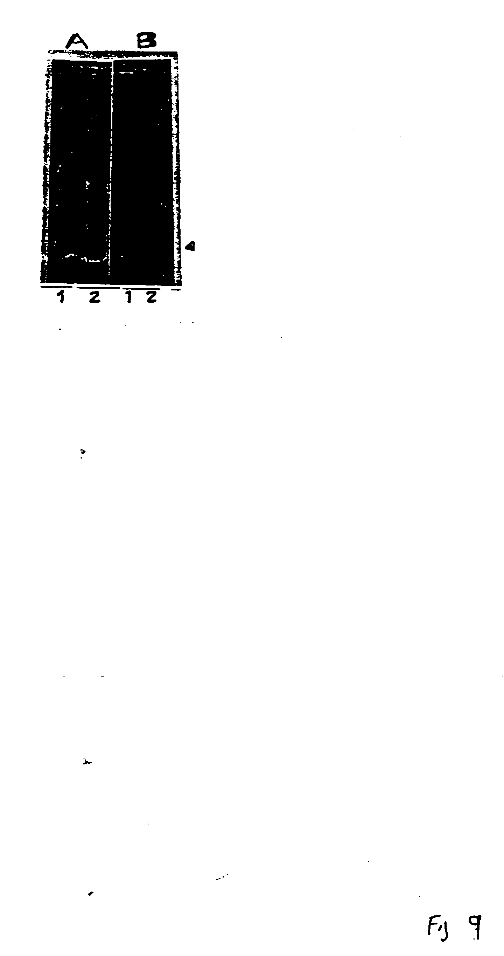

For this purpose, the serum IgE binding of Parietaria was examined in 30 control (skin test negative to grass) and 60 grass allergic (skin test positive) by ELISA (Fig. 8). The results show that Parietaria specific antibodies are present in North American who are skin test positive to pollen allergens. Western blot analysis (Fig. 9) of Parietaria protein extracts using pools of sera from Italy (a pool of 13 sera) and Canada (a pool of 7 sera) showed that a 14 kDa component was recognized by both pools of sera. It can be inferred from these results that antibodies directed against Parietaria can be detected in individuals allergic to pollen in North America.

### SUMMARY OF THE DISCLOSURE

In summary of this disclosure, the present invention provides certain novel nucleic acid molecules which code for allergenic proteins present in Parietaria pollens and used as synthetic peptides having an amino acid sequence corresponding to at least one antigenic determinant of a Parietaria allergen. Modifications are possible within the scope of this invention.

### REFERENCES

1. Firedhoff LR. In: Genetic and Environmental factors in Clinical Allergy, Marsh DG and Blumenthal MN (eds.) Univ. of Minnesota Press (1989).
2. Mohapatra, S.S. In : Clinical Reviews in Allergy, Special topic on "New therapeutic issues on bronchial asthma", T. Nakagawa(ed), Humana press 12:3 (1994).
3. Romagnani, S. Immunology Today 11:316 (1990)
4. Romagnani, S. et al., Ann. Rev. Immunol. 12:227 (1994)
5. Mohapatra, S.S. Pharmacia Allergy Research Foundation Awards Book, PP.4-15, (1992)
6. Loca AF and Cooke RA. J. Immunol. 8: 162 (1923).
7. Richter, M. *et al.* J. Allergy 29: 298 (1958).
8. Marsh, DG. *et al.* Immunology 22: 1013 (1972).
9. Lichtenstein, LM *et al.* In: 11th Int. Congr. of Allergology and Clin. Immunol. Kerr Jw and Ganderton MA (eds.) pp. 285 (1983).
10. Hamilton RG. Curr. Opinions in Immunol. 2: 558 (1990).
11. Bousquet J. *et al.* J. Allergy Clin. Immunol. 84: 546 (1989).
12. Creticos PS *et al*. J. Allergy Clin. Immunol. 84: 197 (1989).
13. Kay AB. Clin. Exp. Allergy 19: 591 (1989).
14. CMS Update: Desensitizing vaccines Brit. Med. J. 293: 948 (1986).
15. Ekramoddoullah AKM. *et al.* Int. Arch. Allergy Clin. Immunol. 80:100 (1986).
16. Kahn CR and Marsh DG. Fed. Proc. 41: 826 (1982).
17. Chua KY. *et al*. J. Exp. Med 167: 175 (1988).
18. Chua KY. *et al.* Int. Arch. Allergy Clin. Immunol. 85: 127 (1988).
19. Tovey ER, Johnson MC, Roche AL, Cobon GS, Baldo BA. J. Exp. Med. 170: 1457 (1989).
20. Fang KSY. *et al*. Proc. Natl. Acad. Sci. USA 85: 895 (1988).
21. Breiteneder H. *et al*. EMBO J. 8: 1935 (1989).
22. Mohapatra SS. *et al*. Int. Arch. Allergy Appl. Immunol. 91: 362 (1990).
23. D'Amato G. *et al*. J. Allergy clin. Immunol. 83: 116-122 (1989).
24. Charpin J and Surinyach R. Atlas of European allergenic pollens (Sandoz, Paris 1974).
25. Corbi AL. and Carreira J. Int. Archs Allergy appl. Immun. 74: 318-323 (1984).
25a. Holgate S.T., et al. Clin. Allergy 18: 549 (1988)
26. Feo D. *et al*. Molec. Immunol. 21: 25-36 (1984).
26a. Kautman H.S., Ann Allergy 64: 293 (1990)
27. Falagiani P. *et al*. J. Chromat. 328: 425-431 (1985).
27a. Andri L. et al. Allergy 47: 318 (1992).
27b. Ortolani et al. Allergy 49: 13 (1994).
28. Corbi AL. *et al*. Molec. Immunol. 22: 1081-1089 (1985).
29. Geraci D. *et al*. Int. Archs Allergy appl. Immun. 78: 421-428 (1985).
30. Corbi AL. *et al*. J. Immunol. Methods 83: 83-88 (1985).
31. Ford SA. *et al*. Int. Archs Allergy appl Immun. 20: 120-126 (1986).
32. Bolzacchini E. *et al*. J. Chromat. 397: 299-306 (1987).
33. Giuliani A. *et al*. Allergy 42: 434-440 (1987).
34. Bassoli A. *et al*. J. Chromat. 44: 209-218 (1988).
35. Corbi AL. *et al*. Int. Archs Allergy appl. Immun. 77: 377-385 (1985).
36. Corbi AL. *et al*. Ann. Allergy 54: 142-147 (1985).
37. Balzacchini E. *et al*. Allergy 43: 53-59 (1988).
38. Ayuso R. *et al*. Molec. Immunol. 25: 49-56 (1988).
39. Cocchiara R. *et al*. Int. Archs Allergy appl. Immun. 90: 84-90 (1989).
40. Polo F. *et al*. Molec. Immunol. 27: 151-157 (1990).
41. Menegozzo M. et al. Immunochemistry 13:475-476 (1976).
42. Geraci D. et al. Immunochemistry 15:491-478 (1978).
43. Ruffilli A. et al. Molec. Immunol. 24:305-312 (1987).
44. Oreste U. et al. Int. Arch. Allergy Appl. Immunol. 96:19-27 (1991).
44a. Costa M.A. et al, FEBS Letters 341: 182 (1984).
45. Mistrello G. et al. Immunology Lett 40:31-36 (1994).
46. O'Hagan (1992) Clin. Pharmokinet. 22:1 (1992).
47. Ulmer et al., (1993) Curr. Opinion Invest. Drugs. 2 (9): 983-989.
48. Chang et al., (1978) Nature 375:615.
49. Itakura et al., (1977) Science 198:1056.
50. Goeddel et al., (1979) Nature 281:544.
51. Goeddel et al., (1980) Nucl. Acids Res. 8:4057.
52. Chirgwin, J.M. et al. Biochemistry 18:5294 (1979).
53. Tabor S and Richardson CC. Proc. Natl. Acad. Sci. U.S.A. 84: 4767 (1987).
54. Kaslow DC. Nucleic Acids Res. 14: 6767 (1986).

**TABLE 1**

| **Relationships among the Parietaria pollen cDNA clones selected from λgt22 library** | |
|---|---|
| Family | Clones |
| PJ001 | PAR 64 |
| | PAR 16 |
| | PAR 5 |
| | PAR 21 |
| | PAR 48 |
| | PAR 39 |
| | PAR 9 |
| PJ002 | PAR 19 |
| | PAR 19.1 |
| PJ003 | PAR 70 |
| | PAR 10 |

**TABLE 2**

| **Synthesis of overlapping peptides of Par 64 protein.** | | |
|---|---|---|
| SEQ ID NO: | Peptide | Position on Par 64 protein |
| 21 | MEGVL YCKPH FEQLF | 1-15 |
| 22 | FEQLF KETGN FNKKF | 11-25 |
| 23 | FNKKF QSGKP PNEQL | 21-35 |
| 24 | PNEQL VRAPS KLSSL | 31-45 |
| 25 | KLSSL FSGTQ EKCAV | 41-55 |
| 26 | EKCAV CTKTV YPLEK | 31-65 |
| 27 | YPLEK VTVEG NFYHK | 61-75 |
| 28 | NFYHK KCFRC FHGRC | 71-85 |
| 29 | FHGRC FLNPS SYAAL | 81-95 |
| 30 | SYAAL DGTLY CKPHF | 91-105 |
| 31 | CKPHF SQLFK EKGSY | 101-115 |
| 32 | EKGSY NHLIT KAASM | 111-125 |
| 33 | KAASM RKNTL YLLST | 121-135 |
| 34 | YLLST PNPTP TNQYL | 131-146 |
| 35 | TNQYL TGGRV | 141-150 |

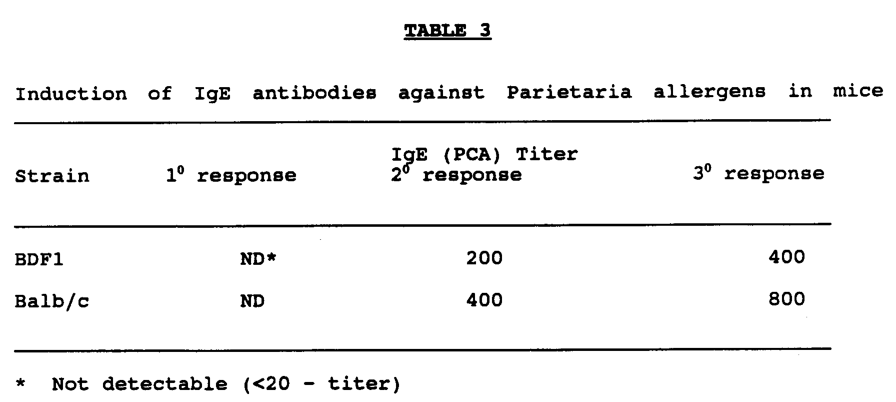

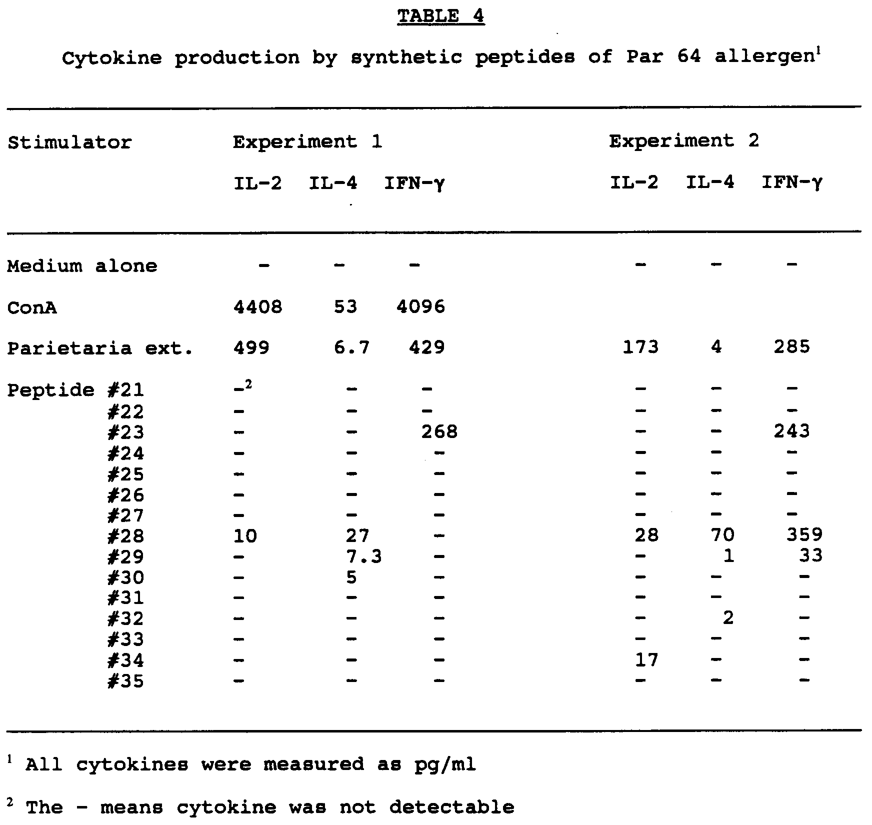

## Claims

1. A purified and isolated nucleic acid molecule, characterized by at least a portion coding for an allergenic protein present in pollen from a plant from the genus Parietaria.

2. The nucleic acid molecule claimed in claim 1, wherein said plant is selected from the family Urticaceae.

3. The nucleic acid molecule claimed in claim 2, wherein said plant is selected from P. judaica and P. officinalis.

4. The nucleic acid molecule claimed in any one of claims 1 to 3, wherein the portion coding for the allergenic protein is:
(A)
(a) a DNA molecule(s) including the DNA sequence(s) for the PJ001 family set out in Figure 3A or its complementary strand;
(b) a DNA molecule(s) encoding a protein(s) from the PJ001 family including the amino acid sequence(s) set out in Figure 3B; or
(c) a DNA molecule(s) which hybridizes under stringent conditions to the DNA molecule(s) defined in (A) (a) or (A)(b);
(B)
(a) a DNA molecule(s) including the DNA sequence(s) for the PJ002 family set out in Figure 3D or its complementary strand;
(b) a DNA molecule(s) encoding a protein(s) from the PJ002 family including the amino acid sequence(s) set out in Figure 3E; or
(c) a DNA molecule(s) which hybridizes under stringent conditions to the DNA molecule(s) defined in
(B) (a) or (B)(b); or
(C)
(a) a DNA molecule(s) including the DNA sequence(s) for the PJ003 family set out in Figure 3F or its complementary strand;
(b) a DNA molecule(s) encoding a protein(s) from the PJ003 family; or
(c) a DNA molecule(s) which hybridizes under stringent conditions to the DNA molecule(s) defined in (C)(a) or (C)(b).

5. The nucleic acid molecule claimed in claim 4 wherein said DNA molecule(s) defined in (A) (c), (B) (c) and (C) (c) have at least about 75% sequence identity with the DNA molecules defined in (A) (a), (B) (a) or (C) (a) or (A) (b), (B) (b) or (C) (b) respectively.

6. A recombinant plasmid adapted for transformation of a host, characterized by a plasmid vector into which has been inserted a DNA segment comprising at least a 15 bp fragment of a nucleic acid molecule as claimed in any one of claims 1 to 5.

7. An expression vector adapted for transformation of a host, characterized by at least a DNA segment comprising at least a 15 bp fragment of a nucleic acid molecule as claimed in any one of claims 1 to 5 and expression means operatively coupled to the DNA segment for expression thereof in the host.

8. The expression vector claimed in claim 7, wherein said DNA segment further comprises a nucleic acid sequence encoding a carrier protein for expressing a carrier-allergen fusion.

9. A recombinant protein produced by expression in said host of the DNA fragment contained in the expression vector claimed in claim 7 or 8 or a functional analog of the protein.

10. A synthetic peptide having an amino acid sequence corresponding to at least one antigenic determinant of a Parietaria allergen.

11. The synthetic peptide claimed in claim 10, wherein said Parietaria allergen which is selected from the PJ001 family, the PJ002 family and the PJ003 family.

12. The synthetic peptide claimed in claims 10 or 11, wherein said at least one antigenic determinant is included within the amino acid sequence FNKKFQSGKPPNEQL, YPLEKVTVEGNFYHKKCFRCFHGRC, YPLEKVTVEGNFYHK or NFYHKKCFRCFHGRC.

13. A composition for protecting allergic individuals from developing an allergic reaction, comprising at least one active component which is at least one nucleic acid molecule as claimed in any one of claims 1 to 5, at least one recombinant protein as claimed in claim 9, or at least one synthetic peptide as claimed in any one of claims 10 to 12, and a pharmaceutically-acceptable carrier therefor.

14. The composition claimed in claim 13 formulated as a vaccine for in vivo administration.

15. The composition claimed in claim 13 or 14, wherein said vaccine comprises said at least one recombinant protein conjugated to a non-immunogenic substrate.

16. The composition claimed in claim 15, wherein said non-immunogenic substrate is potassium cyanate (KCNO) or comprises beads for targetted uptake of said at least one recombinant protein by selected antigen-presenting cells.

17. The composition as claimed in any one of claims 13 to 16 comprising at least one additional desensitizing agent, which is selected from Poa p IX allergen, Lol p I allergen, Bet v I allergen, Amb a I allergen, Amb a II allergen and CRAL 51 allergen.

18. The composition claimed in any one of claims 13 to 17, wherein said at least one nucleic acid molecule is contained in a vector, which is selected from Salmonella, BCG, adenovirus, poxvirus, vaccinia or poliovirus.

19. An antiserum specific for a recombinant protein as claimed in claim 9, a peptide as claimed in any one of claims 10 to 12, or an immunogenic composition as claimed in any one of claims 13 to 18.
